# EUROPÄISCHE PATENTSCHRIFT

(11) **EP 0 265 818 B1**
(45) Veröffentlichungstag und Bekanntmachung des Hinweises auf die Patenterteilung: **28.09.1994**
(21) Anmeldenummer: 87115365.6
(22) Anmeldetag: 21.10.1987
(51) Int. Cl.: C07C 233/47, C07C 275/16, C07C 311/06, C14C 9/00, C11D 1/52, B01F 17/28

(54) **Verfahren zur Herstellung von N,N-disubstituierten Beta-Aminopropionsäuren und ihre Verwendung zur Hydrophobierung von Leder und Pelzen**
Process for the preparation of N,N-disubstituted beta-aminoacids and their use in water-proofing leather and skins
Procédé de préparation d'acides propioniques bêta-aminés N,N-disubstitués et leur utilisation pour l'imperméabilisation du cuir et des peaux

(30) Priorität: 27.10.1986 DE 3636497; 27.05.1987 DE 3717961
(43) Veröffentlichungstag der Anmeldung: 04.05.1988
(73) Patentinhaber: Chemische Fabrik Stockhausen GmbH, D-47805 Krefeld (DE)
(72) Erfinder: Dahmen, Kurt, Dr. Dipl.-Chem., D-4050 Mönchengladbach-Rheydt (DE); Mertens, Richard, Dr. Dipl.-Chem., D-4150 Krefeld (DE); Stockhausen, Dolf, Dr. Dipl.-Kaufm., D-4150 Krefeld (DE)
(74) Vertreter: Klöpsch, Gerald, Dr.-Ing. Patentanwalt

(56) Entgegenhaltungen:
- EP-A- 0 000 494
- BE-A- 445 437
- DE-A- 3 110 646
- GB-A- 1 329 491
- US-A- 3 300 338
- JOURNAL OF MEDICINAL CHEMISTRY; Band 15, Juli 1972, S. 709-13; Z. M. ISRAILI et al: "Metabolites of probenecid, chemical, physical and pharmacological studies"
- JOURNAL OF THE AMERICAN CHEMICAL SOCIETY, Band 91, 10. September 1969, S. 5264-70; Y. KONDO et al: "Selective photoreduction of nucleic acids and their building stones. VIII. Photoreduction and derivation of 1,3-dimethyluracil"
- CHEMICAL ABSTRACTS, Band 95, Nr. 19, 9. November 1981, S. 632, Zusammenfassung Nr. 178604z, Columbus, Ohio, US; Konishiroku Photo Industry Co., Ltd., "Silver halide photographic color developers"; & JP-A-81 40 824
- CHEMICAL ABSTRACTS, Band 67, Nr. 21, 20. November 1967, S. 9459, Zusammenfassung Nr. 100392f, Columbus, Ohio, US; D. GROSS et al: "Synthesis of radioactively labeled compounds. XIX. Synthesis of beta-alanine-3-14C,15N and N-methyl-14C-beta-alanine-3-14C,15N"

## Beschreibung

Die Erfindung betrifft ein Verfahren zur Herstellung von N,N-disubstituierten β-Aminopropionsäuren und ihre Verwendung als Emulgatoren, Netzmittel, Tenside in Reinigungsmitteln und zur Hydrophobierung von Leder und Pelzen, insbesondere ein Verfahren zur Herstellung von N-Alkyl-N(2-carboxyethyl)amiden, neue N-Alkyl-N(2-carboxyethyl)sulfonamide und N-Alkyl-N(2-carboxyethyl)-harnstoffe sowie deren Verwendung.

Alkylaminopropionsäuren, Alkyliminodipropionsäuren sowie die acylierten Alkylaminopropionsäuren sind an sich bekannt. So wird in der US-PS 2.468.012 die Herstellung von N-Alkylaminopropionsäuren und ihre Verwendung als Detergentien, Emulgatoren und Schaumstabilisatoren beschrieben. Die Herstellung erfolgt durch Addition von primären Aminen an Acrylsäureester und Verseifung der entstandenen N-Alkylaminopropionsäureester zu den entsprechenden Säuren bzw. Salzen.

In der DE-OS 20 54 649 werden N-Acyl-N-alkylaminopropionsäuren beschrieben, die durch Umsetzung von Aminen mit Acrylsäurederivaten wie den Acrylsäureestern, Acrylnitril oder Acrylamid und anschließender Reaktion mit Anhydriden hergestellt werden. Im letzten Syntheseschritt wird die Ester-, Nitril- oder Amidfunktion zur Säure verseift. Die Produkte werden als wässrige bzw. wässrig- alkoholische Lösung erhalten.

Diese bekannten Herstellungsverfahren weisen eine Reihe von Nachteilen auf:
Der notwendigerweise erforderliche Verseifungsschritt hat zur Folge, daß die Produkte nur in Lösung erhältlich sind und nicht ohne weitere Verfahrenschritte als 100 %ige Substanz. Durch die Ausbildung von Gelstrukturen sind in Wasser sogar nur 20 bis 30 %ige Lösungen von pastöser Konsistenz erhältlich. Nur durch Mitverwendung von Alkoholen, die nachteilig ist, läßt sich die Konzentration auf etwa 50 % anheben. Da die Verseifung im stark alkalischen Bereich durchgeführt wird, enthalten die Produkte nach der Einstellung auf pH-Werte von 5 bis 8, wie sie für viele Verwendungen z.B. im Textilbereich gefordert werden, hohe Salzgehalte, die jedoch oft unerwünscht sind.

Bei der Verwendung von festen Alkylaminen muß die Addition an das Acrylsäurederivat bei erhöhten Temperaturen (oberhalb des Schmelzpunktes des Alkylamins) durchgeführt werden, wodurch bei den reaktiven Acrylderivaten die unerwünschte Nebenreaktion der Diadduktbildung gefördert wird. Bei dieser Nebenreaktion wird ein zweites Molekül Acrylderivat an schon gebildetes N-Alkylaminopropionsäurederivat addiert, wobei ein tertiäres Amin entsteht, das im folgenden Syntheseschritt nicht mehr acyliert werden kann.

Nachteilig wirkt sich schließlich auch der Geruch von Restgehalten an Acrylester oder Acrylnitril im Produkt aus. So ist ein Restgehalt von 0,1 % Acrylester deutlich wahrzunehmen.

Aufgabe der Erfindung ist die Schaffung eines Verfahrens, das die vorstehend genannten Nachteile nicht aufweist, insbesondere die Herstellung der Verfahrensprodukte in Substanz ohne unerwünscht hohe zusätzliche Salzgehalte und ohne die Bildung von unerwünschten Nebenprodukten ermöglicht.

Gelöst wird diese Aufgabe durch ein Verfahren zur Herstellung von Verbindungen der allgemeinen Formel I 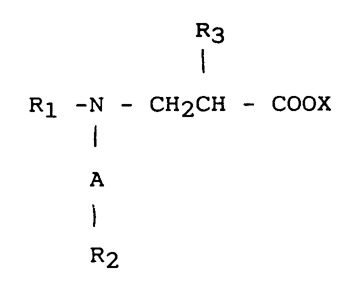
in der
- R₁: einen gesättigten oder ungesättigten Alkylrest oder Alkoxyalkylrest mit 1 - 22 Kohlenstoffatomen, bevorzugt 12 - 18 Kohlenstoffatomen,
- R₂: einen Alkylrest mit 1 - 18 Kohlenstoffatomen, den Oleoylrest, einen gesättigten oder ungesättigten Carboxyalkylrest mit 3 - 4 Kohlenstoffatomen, einen Carboxyphenylrest, einen Carboxylrest oder einen Toluylrest
- R₃: Wasserstoff oder CH₃ und
- X: Wasserstoff, Alkalimetall, Erdalkalimetall, Ammonium, Alkylammonium bzw. Alkanolammonium bedeuten
und
- A: für 〉C=O 〉SO₂ , -CONH- oder einen Alkylenrest mit O-3 C-Atomen
steht, mit der Maßgabe, daß,
1) wenn A für einen Alkylenrest mit 0 - 3 C-Atomen steht, R₂ nicht einen Alkylrest mit 1 - 18 C-Atomen oder den Oleoylrest bedeuten kann,
2) wenn A für 〉SO₂ oder -CONH- steht, R₂ nicht ein gesättigter oder ungesättigter Carboxyalkylrest mit 3 - 4 C-Atomen, ein Carboxyphenylrest oder der Carboxylrest sein kann,
3) wenn A für 〉C=O steht, R₂ nicht der Carboxylrest sein kann,
dadurch gekennzeichnet, daß Alkylamine der Formel R₁-NH₂, worin R₁ die oben beschriebene Bedeutung hat, an (Meth)-acrylsäure angelagert werden und die entstandenen N-Alkylaminopropionsäuren mit Carbonsäureanhydriden, Carbonsäurechloriden, Sulfonsäurechloriden, Isocyanaten, Halogencarbonsäuren oder (Meth-)acrylsäuren umgesetzt und anschließend gegebenenfalls wenigstenteilweise neutralisiert werden.
Überraschenderweise hat sich gezeigt, daß sich Alkylamine bei 40 bis 130°C , vorzugsweise 70 - 100°C , an (Meth)acrylsäure addieren lassen. Obwohl die Reaktionskomponenten nach dem Vermischen als organisches Salz vorliegen, ist die Addition nach 1 - 4 Std. beendet und hat einen vollständigen Umsatz erreicht. Die Diadduktbildung wird bei diesen Reaktionsbedingungen nicht beobachtet. Ebenso ist die Verwendung eines Lösungsmittels nicht erforderlich.

Bei Aminen mit einem Siedepunkt unter den sogenannten Reaktionstemperaturen wird die Umsetzung bevorzugt unter Druck, z.B. im Autoklaven, durchgeführt.

Die Umsetzung von Alkylamin mit (Meth)acrylsäure erfolgt bevorzugt in equimolaren Mengen.

Als Alkylamine der Formel R₁-NH₂ lassen sich z.B. die folgenden primären Amine einsetzen: Methylamin, Propylamin, Butylamin, Hexylamin, Nonylamin, Decylamin, Dodecylamin, Tridecylamin, Isotridecylamin, Hexadecylamin, Octadecylamin, Propoxypropylamin, Octyloxypropylamin, Oleylamin, Oleyloxypropylamin, Laurylamin, Kokosfettamin, Stearylamin, Talgfettamin oder Gemische der o.g. Amine. Laurylamin, Kokosfettamin, Talgfettamin und Oleylamin sind bevorzugte Amine.

Die weitere Umsetzung der N-Alkyl-N(2-carboxyethyl)-amine wird in Substanz mit Anhydriden, Isocyanaten, Carbonsäurechloriden oder Sulfonsäurechloriden durchgeführt, wenn es sich um eine Acylierung handelt. Bei Temperaturen von 50° - 100°C wird das Acylierungsmittel in equimolaren Mengen bis zu einem geringem Überschuß (ca. 5 %) eingesetzt.

Die Reaktion mit Anhydriden oder Isocyanaten ist nach 1 - 2 Stunden beendet und liefert die gewünschten N-Alkyl-N(2-carboxyethyl)amide in 100 %iger Form. Bei der Verwendung von Monocarbonsäureanhydriden,z.B. Essigsäureanhydrid, wird die in äquivalenter Menge anfallende Monocarbonsäure durch Destillation oder Auswaschen mit Wasser abgetrennt.

Als Anhydride können beispielhaft genannt werden:
Essigsäureanhydrid, Propionsäureanhydrid, Buttersäureanhydrid, Maleinsäureanhydrid, Bernsteinsäureanhydrid, Phthalsäureanhydrid.

Als Isocyanate kommen beispielsweise infrage:
Butylisocyanat, Toluylisocyanat, Isopropylisocyanat.

Auch die zweite Stufe, d.h die Acylierungsreaktion wird bevorzugt mit equimolaren Mengen der Reaktionsteilnehmer durchgeführt.

Wird die Acylierungsreaktion mit Carbonsäurechloriden oder Sulfonsäurechloriden durchgeführt, werden als Säurebinder pulverförmige Soda oder tertiäre Amine verwendet. Das während der Reaktion entstandene Salz kann abfiltriert oder mit Wasser ausgewaschen werden.

Als Beispiele für Carbonsäurechloride können genannt werden:
Essigsäurechlorid, Propionsäurechlorid, Ölsäurechlorid und geeignete Sulfonsäurechloride z.B. Methansulfonsäurechlorid, Ethansulfonsäurechlorid, p-Toluolsulfonsäurechlorid.

Die weitere Umsetzung der entstandenen N-Alkylaminopropionsäure kann auch mit Halogencarbonsäuren, bevorzugt Chlorcarbonsäuren, (z.B. Chlorameisensäure, Chloressigsäure, Chlorpropionsäure und Chlorbuttersäure), oder (Meth-)acrylsäure vorgenommen werden.

Durch das neue Verfahren gelingt es, die erfindungsgemäßen N,N-disubstituierten β-Aminopropionsäurederivate, in einem Eintopfverfahren in zwei Syntheseschritten ohne Nebenproduktbildung herzustellen. Die Produkte werden in 100 %iger Form ohne Lösungsmittel erhalten.

Zur Neutralisation werden die N,N-disubstituierten β-Aminopropionsäurederivate mit wäßriger Alkalimetallhydroxid-, Erdalkalimetallhydroxid- oder Ammoniaklösung vermischt. Je nach Konzentration und Neutralisationsmittel werden viskose bis pastöse wäßrige Lösungen erhalten.
In einer bevorzugten Ausführung wird die Neutralisation mit organischen Basen durchgeführt. Als organische Basen kommen besonders primäre, sekundäre und tertiäre Amine in Frage. Beispiel hierfür sind die Alkylamine und Alkanolamine. Besonders bevorzugt sind die Ethanolamine und Isopropanolamine. Das Neutralisationsmittel kann sowohl äquivalent der Menge Säure zugesetzt werden als auch im Unterschuß.

Verwendung finden die N,N-disubstituierten β-Aminopropionsäurederivate und ihre Salze als Emulgatoren und Netzmittel oder als hautschonende Tenside in Reinigungsmitteln. Ein weiteres Einsatzgebiet liegt in Formulierungen für die Lederhydrophobierung. Die Erfindung betrifft somit auch ein Verfahren zum Hydrophobieren von Leder und Pelzen unter Verwendung der erfindungsgemäßen Verbindungen allein oder im Gemisch mit anderen bekannten Hydrophobiermitteln.

Die Herstellung wasserdichten Leders erfolgt im wesentlichen nach folgenden zwei Verfahren:

### 1. Das Füllen der kapillarartigen, wassersaugenden Faserzwischenräume mit wasserabdichtenden Substanzen

Wasserunlösliche Stoffe, wie z.B. Fette, Paraffine, Wachs oder bestimmte Polymere zeigen dabei die unerwünschte Begleiterscheinung, insbesondere die für den Tragekomfort wichtigen Eigenschaften der Luft- und Wasserdampfdurchlässigkeit negativ zu beeinflussen. Günstiger verhalten sich Verbindungen, die durch Wasseraufnahme quellen und damit den Eigenquell- und -abdichtungseffekt der Lederfaser unterstützen. Die nach dieser Methode eingesetzten Produkte gehören zum Typ der Wasser-in-Öl (W/O)-Emulgatoren.

### 2. Die Umhüllung der Faserbündel mit hydrophoben Substanzen

Emulgatoren, die mit mehrwertigen Metallionen (Kationen) Komplexe bilden können, vermögen vielfach auch in den Chrom-Kollagen-Komplex einzutreten und bewirken dann Hydrophobie. Derartige Emulgatoren werden chemisch an die chrom- oder aluminiumgegerbte Lederfaser gebunden und setzen durch diese hydrophobe Umhüllung die Benetzbarkeit und die Wasseraufnahmefähigkeit herab. Die Erniedrigung des Spreitungsvermögens äußert sich in der Bildung kugelförmiger Tropfen (Abperleffekt).

Die in letzter Zeit drastisch gestiegenen Anforderungen an hydrophobierte Leder lassen sich mit den bekannten Hydrophobierungsmethoden kaum oder nur unter Beeinträchtigung anderer auch wichtiger Eigenschaften erzielen. Gebrauchsoberleder sollen heute Anforderungen erfüllen, die vor wenigen Jahren lediglich an Behördenleder (Militärleder) gestellt wurden, ohne jedoch auf Tragekomfort, modisches Erscheinen u.a. zu verzichten.

Aus diesem Grunde ist man beispielsweise auf eine zweistufige Hydrophobierung ausgewichen. Fertiggetrocknete oder zugerichtete Leder lassen sich mit in organischen Lösungsmitteln gelösten Silikonen nachbehandeln. Nachteilig wirkt sich hierbei die zum Vernetzen erforderliche Trocknungstemperatur zwischen 90°C und 100°C aus, die zu erheblichen Maßverlusten führt. Außerdem stellt der hohe Anteil an organischen Lösemitteln, der häufig in die Umwelt entlassen wird, ein Problem dar.
Besonders unerwünscht im Sinne einer rationellen Fertigung ist die Tatsache, daß eine Nachbehandlung mit einem zusätzlichen, unerwünschten Arbeitsschritt verbunden ist.

Es war deshalb auch Aufgabe der Erfindung, ein Verfahren zum Hydrophobieren von Leder und Pelzen bereitzustellen, welches einstufig arbeitet, in wässriger Flotte und im Faß durchgeführt werden kann und mit dem gleichzeitig Fettung und eine massive Hydrophobierung erreicht werden können. Darüberhinaus sollten die verwendeten Hilfsmittel möglichst einfach hergestellt werden können und preiswert sein und ausreichende Fettung und Hydrophobierung nicht nur in einem Behandlungsschritt, sondern auch mit einem einzigen Produkt erreicht werden.

Gelöst wird diese Aufgabe durch ein Verfahren zum Hydrophobieren von Leder und Pelzen, dadurch gekennzeichnet, daß man Verbindungen der allgemeinen Formel I 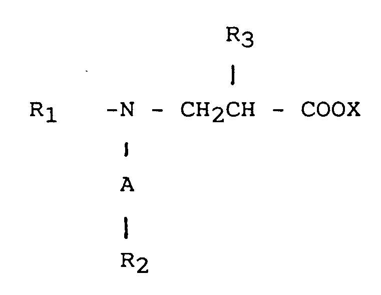
in der
- R₁: einen gesättigten oder ungesättigten Alkylrest oder Alkoxyalkylrest mit 1 - 22 Kohlenstoffatomen, bevorzugt 12 - 18 Kohlenstoffatomen,
- R₂: einen Alkylrest mit 1 - 18 Kohlenstoffatomen, den Oleoylrest, einen gesättigten oder ungesättigten Carboxyalkylrest mit 3 - 4 Kohlenstoffatomen, einen Carboxyphenylrest, einen Carboxylrest (-COOH) oder einen Toluylrest
- R₃: Wasserstoff oder CH₃ und
- X: Wasserstoff, Alkalimetall, Erdalkalimetall, Ammonium, Alkylammonium bzw. Alkanolammonium
bedeuten
und
- A: für 〉C=O, 〉SO₂, -CONH- oder einen Alkylenrest mit O-3 C-Atomen
steht, mit der Maßgabe, daß,
1) wenn A für einen Alkylenrest mit 0 - 3 C-Atomen steht, R₂ nicht einen Alkylrest mit 1 - 18 C-Atomen oder den Oleoylrest bedeuten kann,
2) wenn A für 〉SO₂ oder -CONH- steht, R₂ nicht ein gesättigter oder ungesättigter Carboxyalkylrest mit 3 - 4 C-Atomen, ein Carboxyphenylrest oder der Carboxylrest sein kann,
3) wenn A für 〉C=O steht, R2 nicht ein Carboxylrest sein kann,
in einer Menge von 0,1 - 25 Gew.-%, vorzugsweise 1 bis 15 Gew.-%, bezogen auf das Falzgewicht des Leders oder des Naßgewichts der Pelze, bei pH-Werten von 4 bis 9 auf die zu behandelnden Leder oder Pelze während oder nach der Nachgerbung einwirken läßt, anschließend auf pH-Werte im Bereich von 3,5 bis 5,0 einstellt und nach der Behandlung ggfs. mit einem 2-, 3- oder 4-wertigen Metallsalz, vorzugsweise in wäßriger Lösung, fixiert.

Die erfindungsgemäßen Produkte werden entweder direkt in ihrer wenigstens teilneutralisierten Form als etwas Wasser enthaltendes Produkt von meist pastöser Konsistenz zur Hydrophobierung eingesetzt oder die nicht neutralisierten, meist pastösen Produkte werden in Kohlenwasserstoffen und/oder Chlorkohlenwasserstoffen, gegebenfalls mittels Lösungsvermittlern, gelöst und anschließend wenigstens teilneutralisiert. Hierzu eignen sich die vorstehend erwähnten Basen, insbesondere organische primäre, sekundäre und tertiäre Amine und Alkanolamine, sowie wässrige Lösungen von Ammoniak und Alkalihydroxiden. Die emulgierende Wirkung der neutralisierten Produkte erlaubt auch die Herstellung wasserhaltiger Produkte.

Die erfindungsgemäßen Produkte werden in einer Menge von 0,1 bis 25 vorzugsweise 1 bis 15 Gew-%, bezogen auf Falzgewicht des Leders oder des Naßgewichts der Pelze bei pH-Werten zwischen 4 und 9 eingesetzt, worauf anschließend der pH-Wert auf 3,5 bis 5,0 abgesenkt wird und gegebenenfalls mit einem 2-,3- oder 4-wertigen Metallsalz fixiert wird.

Das erfindungsgemäße zur Hydrophobierung eingesetzte Produkt enthält vorteilhaft 5 bis 80 Gew.-%, bevorzugt 10 - 70 Gew.-% der erfindungsgemäßen Verbindungen der allgemeinen Formel I, gegebenenfalls 20 bis 70 Gew.-%, bevorzugt 30 - 60 Gew.-% an Kohlenwasserstoffen oder/und Chlorkohlenwasserstoffen, gegebenenfalls mit Lösungsvermittlern wie höhersiedenden Produkten aus der Oxosynthese und Rest auf 100 % Wasser.

Obwohl bereits mit den erfindungsgemäßen Verbindungen allein eine ausgezeichnete Fettung und Hydrophobierung erreicht wird, können die erfindungsgemäßen Hydrophobiermittel zusammen mit bekannten Fettungs-und/oder Hydrophobiermitteln wie Phosphorsäureteilestern, Silikonölen, Ölsäuresarkosid und/oder (Chlor-)Paraffinen eingesetzt werden.

Sofern Phosphorsäureteilester mit verwendet werden, werden bevorzugt Teilester von Alkoholen einer Kettenlänge C₄ bis C₂₀, bevorzugt C₁₆-C₁₈, die ggfs. oxalkyliert sind, in Mengen von 0,5 bis 30 Gew.-%, bevorzugt 1-20 Gew.-%, (bezogen auf 100 g Hydrophobierungsmittel) eingesetzt.

Die Mitverwendung saurer Phosphorsäureester kann derart erfolgen, daß sie zusammen mit den erfindungsgemäßen Verbindungen vorgelegt, verdünnt und dann gemeinsam mit diesen neutralisiert werden. Die Phosphorsäureester können auch in bereits neutralisierter Form zum Einsatz gelangen. Der Einsatz von Phosphorsäureestern bietet sich beispielsweise zur Reduzierung des extrahierbaren Fettgehaltes an; dies stellt insbesondere bei Bekleidungsledern wegen der dadurch verbesserten Chemisch-Reinigungs-Beständigkeit einen Vorteil dar.

Sofern zusätzlich zu den erfindungsgemäßen Verbindungen Silikonöle mit verwendet werden, so kommen diese in Mengen von 0,5 bis 80 Gew.-%, bevorzugt 1 -20 Gew.-% (bezogen auf 100 g Hydrophobiermittelformulierung) zur Anwendung.

Als Silikonöle können handelsübliche Polysiloxane wie Dimethyl- oder Methylphenylpolysiloxan eingesetzt werden. Bevorzugt sind Viskositätsbereiche von 100 bis 500 mPa.s. Dabei wird das Silikonöl mit der erfindungsgemäßen Verbindung ggf. gemeinsam mit Kohlenwasserstoffen und/oder Chlorkohlenwasserstoffen oder Lösungsvermittlern vorgelegt. Der Einsatz von Silikonen dient bevorzugt der Erzielung bestimmter erwünschter Oberflächeneffekte wie z.B. der Narbenglätte.

Zur weiteren Verbesserung der Weichheit und um einen schmalzigeren Griff zu erzielen, ist auch die Mitverwendung von Ölsäuresarkosid möglich. Um bestimmte Effekte zu erzielen, kann generell jede Kombination aus Phosphorsäurepartialester, Silikonöl und Ölsäuresarkosid mit den erfindungsgemäßen Verbindungen eingesetzt werden.

Ölsäuresarkosid kann zusätzlich zu den erfindungsgemäßen Verbindungen in Mengen von 0,5 bis 50 Gew.-%, bevorzugt 2-20 Gew.-% (bezogen auf 100 g der eingesetzten Formulierung) verwendet werden.

Die Erfindung wird durch die folgenden Beispiele erläutert:

### Beispiel 1:

In einem Kolben mit Rührer, Thermometer und Kühler werden 534 g (2 Mol) Oleylamin vorgelegt und auf 60°C erwärmt. Innerhalb von 30 Minuten werden 144 g (2 Mol) Acrylsäure zugetropft, wobei die Temperatur auf ca. 90°C ansteigt. Anschließend wird für weitere 2,5 Stunden bei 90°C gerührt. Durch Bestimmung der primären Basen wird ein vollständiger Umsatz festgestellt. Das Reaktionsprodukt wird auf 70°C abgekühlt und portionsweise mit 196 g (2 Mol) Maleinsäureanhydrid versetzt. Zur Vervollständigung der Reaktion wird 2 Stunden bei 70 - 80°C weitergerührt. Man erhält des N-Oleyl-N(2-carboxyethyl)maleinsäuremonoamid als hellbraune Paste.

| | |
|---|---|
| Säurezahl | 250 mg KOH/g |
| Restgehalt an basischem Stickstoff | 0,2 mmol/g |

Zur Neutralisation wurden bei 80°C unter Rühren 122 g (2 Mol) Monoethanolamin zugetropft.

### Beispiel 2:

Entsprechend Beispiel 1 werden 269 g (1 Mol) Stearylamin an 72 g (1 Mol) Acrylsäure addiert und das Reaktionsprodukt mit 98 g (1 Mol) Maleinsäureanhydrid umgesetzt. Das N-Stearyl-N(2-carboxyethyl)maleinsäuremonoamid erstarrt bei Raumtemperatur zu einer hellbraunen Paste.

| | |
|---|---|
| Säurezahl: | 251 mg KOH/g |
| Restgehalt an basischem Stickstoff: | 0,32 mmol/g |

Es wurden bei 100°C mit 37,5 g (0,5 Mol) Isopropanolamin neutralisiert.

### Beispiel 3:

Entsprechend Beispiel 1 werden 486 g (2,0 Mol) Lauryloxypropylamin an 144 g Acrylsäure (2,0 Mol) addiert und das Reaktionsprodukt mit 196 g (2 Mol) Maleinsäureanhydrid umgesetzt. Das N-Lauryloxypropyl-N(2-carboxyethyl)maleinsäuremonoamid erstarrt bei Raumtemperatur zu einer dunkelbraunen, leicht trüben Paste.

| | |
|---|---|
| Säurezahl | 265 mg KOH/g |
| Restgehalt an basischem Stickstoff: | 0,3 mmol/g |

### Beispiel 4:

Entsprechend Beispiel 1 werden 785 g Cocosfettamin (3,72 Mol; Gehalt an basischem Stickstoff: 4,74 mmol/g) an 268 g Acrylsäure (3,72 Mol) addiert und das Reaktionsprodukt mit 364,8 g (3,72 Mol) Maleinsäureanhydrid umgesetzt. Man erhält ein rotbraunes, hochviskoses Öl.

| | |
|---|---|
| Säurezahl | 290 mg KOH/g |
| Restgehalt an basischem Stickstoff: | 0,25 mmol/g |

Zur Neutralisation wurden bei 80°C unter Rühren 554 g (3,72 Mol) Triethanolamin zugetropft.

### Beispiel 5:

Entsprechend Beispiel 1 werden 101 g (1,0 Mol) Hexylamin an 72 g (1,0 Mol) Acrylsäure addiert und das Reaktionsprodukt mit 98 g (1,0 Mol) Maleinsäureanhydrid umgesetzt. Man erhält das N-Hexyl-N(2-carboxyethyl)maleinsäuremonoamid als hellbraunes Öl.

| | |
|---|---|
| Säurezahl | 418 mg KOH/g |
| Restgehalt an basischem Stickstoff: | 0,15 mmol/g |

### Beispiel 6:

534 g (2 Mol) Oleylamin werden in einem Kolben, der mit Rührer, Thermometer und Kühler ausgerüstet ist auf 60°C erwärmt. Innerhalb von 30 Minuten werden 172 g (2 Mol) Methacrylsäure zugetropft, wobei die Temperatur auf ca. 85°C ansteigt. Zur vollständigen Reaktion wird die Mischung 3 h bei 100°C gerührt. Durch Bestimmung der primären Basen wird ein vollständiger Umsatz festgestellt. Anschließend wird auf 70°C abgekühlt und bei 70 - 80°C 204 g (2 Mol) Essigsäureanhydrid zugetropft. Nach weiteren 2 h wird die entstandene Essigsäure bei einem Vakuum von 20 mbar abdestilliert. Man erhält das N-Oleyl-N(2-carboxy-2-methylethyl)acetamid als braunes, klares Öl.

| | |
|---|---|
| Säurezahl: | 139 mg KOH/g |
| Restgehalt an basischem Stickstoff: | 0,1 mmol/g |

Es wurden bei 80°C mit 112,2 g (2 Mol) KOH, gelöst in 2492 g Wasser, neutralisiert. Man erhält eine wäßrige Lösung mit 25 % Feststoffanteil.

### Beispiel 7:

Analog Beispiel 1 werden 267 g (1,0 Mol) Oleylamin an 72 g (1,0 Mol) Acrylsäure addiert. Anschließend werden bei 90°C 148 g (1,0 Mol) Phthalsäureanhydrid portionsweise eingetragen und für 3 h bei 100°C gerührt. Das N-Oleyl-N-(2-carboxyethyl)phthalsäuremonoamid ist eine gelbbraune Paste.

| | |
|---|---|
| Säurezahl: | 225 mg KOH/g |
| Restgehalt an basischem Stickstoff: | 0,5 mmol/g |

### Beispiel 8:

Analog Beispiel 1 werden 267 g Oleylamin an 72 g Acrylsäure addiert. Das Additionsprodukt wird mit 55 g wasserfreiem Natriumcarbonat vermischt, und bei 100°C werden 301 g (1,0 Mol) Ölsäurechlorid in ca. 2 h zugetropft. Nach 2 weiteren Stunden ist die Acylierung beendet. Das gebildete Kochsalz wird mit Wasser ausgewaschen.

| | |
|---|---|
| Säurezahl: | 94 mg KOH/g |
| Restgehalt an basischem Stickstoff: | 0,1 mmol/g |

### Beispiel 9:

Analog Beispiel 6 werden 267 g (1,0 Mol) Oleylamin an 86 g (1,0 Mol) Methacrylsäure addiert.
Nach der Addition gibt man zur Reaktionsmischung 101 g (1,0 Mol) Triethylamin und fügt portionsweise bei 70 - 90°C 190,5 g (1,0 Mol) p-Toluolsulfonsäurechlorid zu. Nach Abklingen der exothermen Reaktion wird 2 h bei 90°C gerührt und anschließend das Salz mit Wasser ausgewaschen. Das N-Oleyl-N(2-carboxy-2-methylethyl)p--toluolsulfonsäureamid wird als dunkelbraunes, viskoses Öl erhalten.

| | |
|---|---|
| Säurezahl: | 99 mg KOH/g |
| Restgehalt an basischem Stickstoff: | 0 mmol/g |

| | | |
|---|---|---|
| IR: | C = 0 | 1720 cm⁻¹ |
| | C = C | 1620 cm⁻¹ (Aromat) |
| | SO₂ - N | 1330 cm⁻¹ und 1170 cm⁻¹ |

H-NMR: δ 7 - 8 ppm aromat. Protonen
Zur Neutralisation gibt man bei 90°C unter Rühren 105 g (1,0 Mol) Diethanolamin zu.

### Beispiel 10:

Analog Beispiel 1 werden 267 g (1,0 Mol) Oleylamin an 72 g (1,0 Mol) Acrylsäure addiert.
Nach der Addition werden 53 g (0,5 Mol) pulverförmiges Natriumcarbonat zugegeben und bei 110°C 114,5 g (1,0 Mol) Methansulfosäurechlorid zugetropft. Zur Nachreaktion wird weitere 2 h bei 110 °C gerührt. Das enstandene Salz wird mit Wasser ausgewaschen. Man erhält das N-Oleyl-N(2-carboxyethyl)methansulfosäureamid als dunkelbraune Paste.

| | |
|---|---|
| Säurezahl: | 129 mg KOH/g |
| Restgehalt an basischem Stickstoff: | 0 mmol/g |

| | | |
|---|---|---|
| IR: | C = O | 1730 cm⁻¹ |
| | SO₂-N | 1320 cm⁻¹ und 1160 cm⁻¹ |

### Beispiel 11:

Analog Beispiel 1 werden 267 g (1,0 Mol) Oleylamin an 72 g (1,0 Mol) Acrylsäure addiert.
Nach der Addition werden 115 g (1,0 Mol) N-Ethylmorpholin zugegeben und bei 40 - 50 °C 133 g (1,0 Mol) Toluylisocyanat zugetropft. Man läßt 1 h nachreagieren und erhält N-Oleyl-N(2-carboxyethyl)-Nʹ-toluylharnstoff als braunes Öl.

| | |
|---|---|
| Säurezahl: | 89 mg KOH/g |

| | | |
|---|---|---|
| IR: | C = O (Säure) | 1720 cm⁻¹ |
| | N-C=O | 1660 cm⁻¹ und 1560 cm⁻¹ |
| | C = C (Aromat) | 1590 cm⁻¹ und 1610 cm⁻¹ |

### Beispiel 12:

Analog Beispiel 1 werden 267 g (1,0 Mol) Oleylamin an 72 g (1,0 Mol) Acrylsäure addiert.
Nach der Addition werden 115 g (1,0 Mol) N-Ethylmorpholin zugegeben und bei 40 - 50 °C 99 g (1,0 Mol) n-Butylisocyanat zugetropft. Man läßt 1 h nachreagieren und erhält N-Oleyl-N(2-carboxyethyl)-Nʹ-butylharnstoff als hellbraunes Öl.

| | |
|---|---|
| Säurezahl: | 96,4 mg KOH/g |

| | | |
|---|---|---|
| IR: | C = O (Säure) | 1720 cm⁻¹ |
| | N-C=O | 1620 cm⁻¹ und 1540 cm⁻¹ |

### Beispiel 13:

Analog Beispiel 1 werden 204 g Oleylaminopropionsäure (0,6 Mol), 70 g Chloressigsäure (Natriumsalz; 0,6 Mol), 54 g Wasser und 108 g Isopropanol vorgelegt und auf 80 °C erwärmt, anschließend liegt eine klare Lösung vor. Zu dieser Lösung werden 106,6 g 45 %ige Natronlauge (1,2 Mol) innerhalb von 45 Min. zugetropft, wobei die Viskosität der Reaktionsmischung ansteigt. Es wird 4 Stunden bei 82 °C nachgerührt, dann mit konzentrierter Salzsäure neutralisiert und in überschüssiges Wasser gegossen, wobei das Produkt ausfällt. Das Produkt (N-Oleyl-N-carboxymethyl-aminopropionsäure) wird abgetrennt und getrocknet.

| | |
|---|---|
| Säurezahl: | 245 mg KOH/g |

Das als Ausgangsprodukte eingesetzte Additionsprodukt wird analog Beispiel 1 aus der Umsetzung von Oleylamin mit Acrylsäure in equimolaren Mengen erhalten.

### Beispiel 14:

267,5 g Oleylamin (1,0 Mol) und 0,2 g HQME (Hydroquinonmonomethylether) werden vorgelegt. Hierzu werden unter Kühlen bis zu einer Temperatur von maximal 70 °C 144 g Acrylsäure (2,0 Mol) zugetropft, worauf anschließend auf 140 °C erwärmt wird. Ab 80 °C verläuft die Reaktion exotherm und die Viskosität steigt stark an. Nach ca. 10 Min. bei 120 °C wird die Mischung pastös. Es wird dann noch 3 Std. bei 140 °C gerührt. Das Produkt (N-Oleyl-N(2-carboxyethylaminopropionsäure) weist eine Säurezahl von 253 mg KOH/g auf.

### Beispiel 15:

a) In einem Rührgefäß werden 13,6 g Wasser und 22,7 g Natronlauge -45 %ig vorgelegt und auf ca. 50 °C erwärmt. Unter Rühren werden anschließend 63,7 g N-Oleyl-N (2-carboxyethyl)maleinsäuremonoamid von Beispiel 1 zugesetzt, danach homogen verrührt
b) Das Produkt aus a) wurde in die nachfolgende Rezeptur zur Herstellung von Waterproof-Leder eingesetzt und die wasserabweisende Wirkung untersucht:
   Rindleder:
   Typ: Waterproof
   Material:
   Rindhäute, wet blue
   Falzstärke: 2,0 mm
   Prozentagaben bezogen auf Falzgewicht
   Waschen:
   300 % Wasser 35 °C
   + 0,5 % Essigsäure 1:5* 15 Min.
   *1 Vol.-Teil konz.Essigsäure und 5 Vol.-Teile Wasser
   Flotte ablassen
   Waschen:
   300 % Wasser 35 °C 10 Min.
   Flotte ablassen
   Neutralisation/Färbung/Nachgerbung:
   150 % Wasser 35 °C
   2 % Na-Formiat ungelöst 90 Min.
   + 2 % Farbstoff ungelöst 15 Min.
   + 4 % Tanigan QF**ungelöst30 Min.
   + 12 % Kastanie N ungelöst60 Min.
   bei 8 Umdrehungen/Min. über Nacht
   + 250 % Wasser 60 °C 10 Min.
   Flotte ablassen
   Waschen:
   300 % Wasser 60 °C 10 Min.
   Flotte ablassen
   Hydrophobierung:
   100 % Wasser 60 °C
   + 0,5 % Ammoniak 1:5* 2 Min.
   * 1 Vol.-Teil konz.Ammoniak und
   5 Vol.-Teile Wasser
   + 6,3 % des erfindungsgemäßen Hydrophobierungsmittels gemäß Beispiel 15 a
   1:5* 60 Min.
   *1Vol.-Teil Produkt und 5 Vol.-Teile Wasser
   + 0,5 % Ameisensäure 1:5 15 Min. pH: 3,6 - 4,0
   Flotte ablassen
   2 X Waschen:
   300 % Wasser 40 °C je 10 Min.
   Flotte ablassen
   Fixierung:
   150 % Wasser 40 °C
   3 % Baychrom A**ungelöst 60 Min.
   pH: 4,2 - 4,8
   Flotte ablassen
   3 X Waschen:
   300 % Wasser 20 °C je 10 Min.
   Flotte ablassen
   Leder über Nacht auf Bock, abwelken, ausrecken, vakuumtrocknen (80 °C/ 2 Min.), ablüften, klimatisieren, stollen, bügeln (80 °C/0,5 Min.)
   Bally-Penetrometer:
   (10 % Stauchung)
   Wasserdurchtrittszeit:
   > 7/ > 7 Std.
   Wasseraufnahme in %
   nach 7 Std: 9,9 /10,4
   Maeser
   Knickungen: 21924/19207
   Wasseraufnahme in %
   nach Knickungen: 8,0/7,1

**) Produkte der Fa. BAYER AG, Leverkusen

### Beispiel 16:

Es wurde das gemäß Beispiel 1 hergestellte Amid verwendet. In einem Rührgefäß werden 30 g Wasser und 7 g Diisopropanolamin bei ca. 50 °C vorgelegt, bevor unter Rühren die organische Phase bestehend aus 14 g des erfindungsgemäßen Amids, 3,5 g Phosphorsäureester-Aminsalz (hergestelt durch Umsetzung von Alfol 16-18, einem Gemisch von Fettalkoholen aus der Ziegler-Synthese, mit P₂O₅, Molverhältnis 3:1, Diisopropanolamin zur Neutralisation) und 45,5 g Chlorparaffin mit 40 % Kettenchlor eingetragen und homogen verrührt wird.

Diese Formulierung wurde in nachfolgender Rezeptur zur Herstellung von Militärleder eingesetzt:
Rindleder
Typ: Militärleder
Material:
Rindhaut, wet blue
Falzstärke: 2,0 mm
Prozentangaben bezogen auf Falzgewicht
Waschen:
300 % Wasser 35 °C
0,5 % Ameisensäure 1:5* 15 Min.
* 1 Vol.Teil konz.Säure mit 5 Vol.Teile
Wasser
Flotte ablassen
Neutralisation:
100 % Wasser 35 °C
3 % Na-Formiat ungelöst 15 Min.
pH: 4,4
+ 0,5 % Na-Bicarbonat 1:10 90 Min.
pH: 4,8
Flotte ablassen
Waschen:
300 % Wasser 30 °C 10 Min.
Flotte ablassen
Nachgerbung:
200 % Wasser 30 °C
1 % Farbstoff 1:20 20 Min.
+ 4 % Tanigan**QF ungelöst 30 Min.
+ 12 % Quebracho ungelöst 60 Min.
Automatik über Nacht
Ruhen/Laufen 15 Min./1 Min.
+ 200 % Wasser 60 °C 20 Min.
Flotte ablassen
Waschen:
300 % Wasser 60 °C 10 Min.
Flotte ablassen
Hydrophobierung:
200 % Wasser 60 °C
10 % Hydrophobierungsmittel gemäß
Bsp. 16
verdünnt mit Wasser im Verhältnis 1:5 (Vol/Vol) 60 Min.
+ 1 % Ameisensäure 1:5* 15 Min.
+1Vol.Teil konz.Säure und 5 Vol.Teile
Wasser
+ 3 % Baychrom**F ungelöst 60 Min.
pH: 3,6 - 3,7
Flotte ablassen
3 X Waschen:
300 % Wasser 25 °C je 10 Min.
Flotte ablassen
Spülen:
**Wasser** 20°C 10 Min.
** Produkte der Fa. BAYER AG, Leverkusen

Leder über Nacht auf Bock, abwelken, ausrecken, vakuumtrocknen (80 °C/3 Min.), ablüften, stollen, bügeln (80 °C/0,5 Min.).
Bally-Penetrometer
(10 % Stauchung)
Wasserdurchtrittszeit:
> 420/ > 420 Min.
Wasseraufnahme in %:
nach 7 Std.: 12/14
Maeser
Knickungen: 29785/25971
Wasseraufnahme in %
nach Knickungen: 10/12

### Beispiel 17:

a) Man legt 30,5 g Wasser und 5,5 g Natronlauge 45 %ig in einem Rührgefäß bei ca. 50 °C vor. Die organische Phase, bestehend aus 18 g des Amides von Beispiel 1, 40 g eines höhersiedenden Mineralöles und 6 g Dimethylpolysiloxan mit einer Viskosität von 350 mPa.s, wird unter Rühren langsam eingetragen, anschließend bis zur Homogenität verrührt.
b) Die Formulierung aus a) wurde in die Rezeptur gemäß Beispiel 16 eingesetzt und führte zu folgenden Ergebnissen:
   Bally-Penetrometer:
   (10 % Stauchung)
   Wasserdurchtrittszeit:
   > 420/ > 420 Min.
   Wasseraufnahme in %
   nach 7 Std.: 7/8
   Maeser:
   Knickungen: > 48123/ >48316
   Wasseraufnahme in %
   nach Knickungen: 9/8

### Beispiel 18:

Es wurde das erfindungsgemäße Amid aus Beispiel 1 verwendet.

In einem Rührgefäß legt man 39 g Wasser mit 3 g Natronlauge 45 %ig bei ca. 50 °C vor. Danach wird die organische Phase aus 6 g des Amids von Beispiel 1, 6 g Ölsäuresarkosid, 40 g eines höhersiedenden Mineralöles und 5 g Dimethylpolysiloxan einer Viskosität von 350 mPa.s langsam unter Rühren eingetragen und homogenisiert.

Das so erhaltene Produkt wird in die Rezeptur für Waterproof gemäß Beispiel 16 b) eingesetzt, wobei folgende Werte erhalten wurden:
Bally-Penetrometer
(10 % Stauchung)
Wasserdurchtrittszeit:
> 420/ > 420
Wasseraufnahme in %
nach 7 Std. 9/10
Maeser:
Knickungen: 29885/30147
Wasseraufnahme in %:
nach Knickungen: 14/11,6

### Beispiel 19:

a) In einem Rührgefäß werden 39 g Wasser und 4 g Natronlauge 45 %ig bei ca. 50 °C vorgelegt. Unter Rühren wird die organische Phase, bestehend aus 12 g Amid gemäß Beispiel 4, 35 g höhersiedendem Kohlenwasserstoff, 5 g Dimethylpolysiloxan der Viskosität 100 mPa.s und 5 g Phosphorsäurepartialester (Alfol 12-18, umgesetzt mit P₂O₅, Molverhältnis 3:1), eingetragen und homogen gerührt.
b) In die Rezeptur gemäß Beispiel 16 eingesetzt, wurden folgende Werte erzielt:
   Bally-Penetrometer
   (10 % Stauchung)
   Wasserdurchtrittszeit:
   > 7/ > 7 Std.
   Wasseraufnahme in %
   nach 7 Std.: 13,3/12,0
   Maeser
   Knickungen: 15849/16717
   Wasseraufnahme in %
   nach Knickungen: 12/8

### Beispiel 20:

Es wurde das in Beispiel 13 beschriebene Produkt verwendet. Hierzu wurden 20 g dieses Stoffs mit 68,4 g eines Chlorparaffins mit 40 % Kettenchlor in einem Rührgefäß vorgelegt und bei 100 - 105 °C homogen gerührt. Nach Abkühlen auf ca. 70°C wurden dem Gemisch 11,6 g Diisopropanolamin zur Neutralisation
zugesetzt.

Diese Formulierung wurde in nachfolgender Rezeptur zur Herstellung von Waterproof -Leder eingesetzt und auf seine Wirkung untersucht:
Material:
Rindhäute, wet-blue
Falzstärke: ca. 2,0 mm
Prozentangaben bezogen auf Falzgewicht
Waschen:
300 % Wasser 35°C
0,5 % Essigsäure 1:5 15 Min.
Flotte ablassen
Neutralisation:
100 % Wasser 35°C
2 % Na-Formiat ungelöst 15 Min.
+ 0,5 % Na-Bicarbonat 1:10 90 Min.
Flotten-pH: 5,3
Lederquerschnitt gegen Bromkresolgrün:blau
Flotte ablassen
Waschen:
300 % Wasser 30°C 10 Min.
Flotte ablassen
Färbung/Nachgerbung:
100 % Wasser 30°C
1 % Chromlederechtschwarz TU 141 %
1:20 20 Min.
+ 4 % Tanigan QF** ungelöst 30 Min.
+ 12 % Quebracho ungelöst
bei 6 Umdrehungen/Min. über Nacht
**durchgegerbt**
+ 200 % Wasser 60°C 20 Min.
Flotte ablassen
Waschen:
300 % Wasser 60°C 10 Min.
Flotte ablassen
Hydrophobierung:
100 % Wasser 60°C
Fixierung:
8 % Hydrophobiermittel 1:4 60 Min.
+ 1 % Ameisensäure 1:5 15 Min.
+ 3 % Baychrom F 60 Min.
Flotten-pH: 3,6 - 3,7
Flotte ablassen
3 X Waschen:
300 % Wasser 25 °C je 10 Min.
Flotte ablassen
Leder über Nacht auf Bock, abwelken, ausrecken, vakuumtrocknen (80 - 85°C, 2 Min.), ablüften, anfeuchten, stollen, vakuumbügeln (80 - 85°C, 30 Sek.). Während der Naßarbeiten wurden keine außergewöhnlichen Beochtungen gemacht.
Bally-Penetrometer:
(10 % Stauchung)
Wasserdurchtrittszeit:
> 7/ > 7 Std.
Wasseraufnahme in %:
nach 7 Std. 13,1/ 8,2
Maeser:
Knickungen 15407/14320
Wasseraufnahme in %
7,7/5,0
nach Knickungen

## Patentansprüche

1. Verfahren zur Herstellung von Verbindungen der allgemeinen Formel I 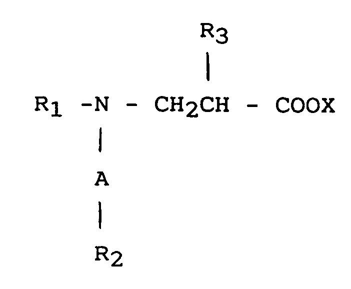 in der
R₁ einen gesättigten oder ungesättigten Alkylrest oder Alkoxyalkylrest mit 1 - 22 Kohlenstoffatomen, bevorzugt 12 - 18 Kohlenstoffatomen,
R₂ einen Alkylrest mit 1 - 18 Kohlenstoffatomen, den Oleoylrest, einen gesättigten oder ungesättigten Carboxyalkylrest mit 3 - 4 Kohlenstoffatomen, einen Carboxyphenylrest, einen Carboxylrest oder einen Toluylrest
R₃ Wasserstoff oder CH₃ und
X Wasserstoff, Alkalimetall, Erdalkalimetall, Ammonium, Alkylammonium bzw. Alkanolammonium
bedeuten
und
A für 〉C=O, 〉SO₂ , -CONH- oder einen Alkylenrest mit O-3 C-Atomen
steht, mit der Maßgabe, daß,
1) wenn A für einen Alkylenrest mit 0 - 3 C-Atomen steht, R₂ nicht einen Alkylrest mit 1 - 18 C-Atomen oder den Oleoylrest bedeuten kann,
2) wenn A für 〉SO₂ oder -CONH- steht, R₂ nicht ein gesättigter oder ungesättigter Carboxyalkylrest mit 3 - 4 C-Atomen oder ein Carboxyphenylrest oder ein Carboxylrest sein kann,
3) wenn A für 〉C=O steht, R₂ nicht ein Carboxylrest sein kann,
dadurch gekennzeichnet, daß Alkylamine der Formel R₁-NH₂, worin R₁ die oben beschriebene Bedeutung hat, bei Temperaturen von 40 - 130 °C an (Meth)-acrylsäure angelagert werden und die entstandenen N-Alkylaminopropionsäuren mit Carbonsäureanhydriden, Carbonsäurechloriden, Sulfonsäurechloriden, Isocyanaten, Halogencarbonsäuren oder (Meth)-Acrylsäuren umgesetzt und anschließend gegebenenfalls wenigstens teilweise neutralisiert werden.

2. Verfahren nach Anspruch 1, dadurch gekennzeichnet, daß die Anlagerung von Alkylaminen an (Meth)acrylsäure bei Temperaturen von 70° - 100°C, durchgeführt wird.

3. Verfahren nach den Ansprüche 1 und 2, dadurch gekennzeichnet, daß Alkylamine und Methacrylsäure in equimolaren Mengen umgesetzt werden.

4. Verfahren nach einem der Ansprüche 1 bis 3, dadurch gekennzeichnet, daß bei Verwendung von Alkylaminen, die unterhalb der Reaktionstemperaturen sieden, die Umsetzung unter Druck durchgeführt wird.

5. Verfahren nach einem der Ansprüche 1 bis 4, dadurch gekennzeichnet, daß die in der ersten Stufe entstandenen N-Alkylaminopropionsäuren bei Temperaturen von 50 bis 100°C mit Carbonsäureanhydriden, Carbonsäurechloriden, Sulfonsäurechloriden, Isocyanaten, Halogencarbonsäure oder (Meth-)acrylsäuren umgesetzt werden.

6. Verfahren nach einem der Ansprüche 1 bis 5, dadurch gekennzeichnet, daß Carbonsäureanhydrid, Carbonsäurechlorid, Sulfonsäurechlorid, Isocyanat, Halogencarbonsäure oder (Meth-)acrylsäuren in equimolarem Verhältnis oder in geringem Überschuß, vorzugsweise bis zu 5 %, zur N-Alkylaminopropionsäure eingesetzt wird.

7. Verfahren nach einem der Ansprüche 1 bis 6, dadurch gekennzeichnet, daß bei Verwendung von Carbonsäurechloriden, Sulfonsäurechloriden oder Halogencarbonsäuren für die zweite Stufe der Reaktion Säure bindende Mittel, bevorzugt pulverförmiges Natriumcarbonat oder tertiäre Amine, zugesetzt werden.

8. Verfahren nach einem der Ansprüche 1 bis 7, dadurch gekennzeichnet, daß die Neutralisation mit organischen Basen, bevorzugt Alkylaminen und Alkanolaminen, insbesondere Ethanolaminen oder Propanolaminen durchgeführt wird.

9. N-Alkyl-N(2-carboxyethyl)sulfonamide oder Harnstoffe der allgemeinen Formel I 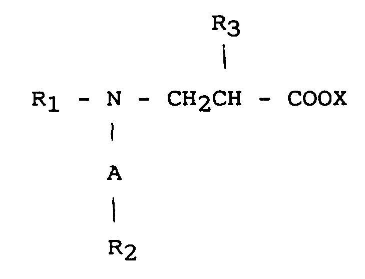 worin R₁, R₂, R₃ und X die in Anspruch 1 genannte Bedeutung haben, dadurch gekennzeichnet, daß A für SO₂= oder -CONH- steht, mit der Maßgabe, daß
R₂ nicht einen Alkylrest mit 1 - 18 C-Atomen, einen gesättigten oder ungesättigten Carboxyalkylrest mit 3 - 4 C-Atomen, ein Carboxyphenylrest, ein Carboxylrest oder ein Toluylrest sein kann.

10. Verfahren zum Hydrophobieren von Leder und Pelzen, dadurch gekennzeichnet, daß man Verbindungen der allgemeinen Formel I 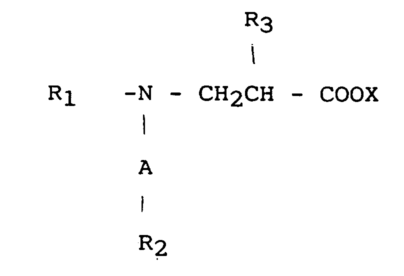 in der
R₁ einen gesättigten oder ungesättigten Alkylrest oder Alkoxyalkylrest mit 1 - 22 Kohlenstoffatomen, bevorzugt 12 - 18 Kohlenstoffatomen,
R₂ einen Alkylrest mit 1 - 18 Kohlenstoffatomen, den Oleoylrest, einen gesättigten oder ungesättigten Carboxyalkylrest mit 3 - 4 Kohlenstoffatomen, einen Carboxyphenylrest einen Carboxylrest oder einen Toluylrest
R₃ Wasserstoff oder CH₃ und
X Wasserstoff, Alkalimetall, Erdalkalimetall, Ammonium, Alkylammonium bzw. Alkanolammonium
bedeuten
und
A für 〉C=O 〉SO₂, -CONH- oder einen Alkylenrest mit O-3 C-Atomen
steht, mit der Maßgabe, daß,
1) wenn A für einen Alkylenrest mit 0 - 3 C-Atomen steht, R₂ nicht einen Alkylrest mit 1 - 18 C-Atomen oder den Oleoylrest bedeuten kann,
2) wenn A für 〉SO₂ oder -CONH- steht, R₂ nicht ein gesättigter oder ungesättigter Carboxyalkylrest mit 3 - 4 C-Atomen oder ein Carboxyphenylrest oder ein Carboxylrest sein kann,
3) wenn A für 〉C=O steht, R₂ nicht ein Carboxylrest sein kann,
in einer Menge von 0,1 - 25 Gew.-% vorzugsweise 1 bis 15 Gew.-% bezogen auf das Falzgewicht des Leders oder des Naßgewichts der Pelze, bei pH-Werten von 4 bis 9 auf die zu behandelnden Leder oder Pelze während oder nach der Nachgerbung einwirken läßt, anschließend auf pH-Werte im Bereich von 3,5 bis 5,0 einstellt und nach der Behandlung ggfs. mit einem 2-, 3- oder 4-wertigen Metallsalz, vorzugsweise in wäßriger Lösung, fixiert.

11. Verfahren nach Anspruch 10, dadurch gekennzeichnet, daß man zur Hydrophobierung ein Produkt einsetzt, welches 5-80, bevorzugt 10 -70 Gew.-% an Verbindungen der allgemeinen Formel I, bevorzugt in wenigstens teilneutralisierter Form, gffs.20-70, bevorzugt 30 - 60 Gew.-% an Kohlenwasserstoffen, ggfs. Lösungsvermittler, sowie Rest auf 100 % Wasser enthält.

12. Verfahren nach einem der Ansprüche 10 bis 11, dadurch gekennzeichnet, daß das eingesetzte Hydrophobiermittel Phosphorsäureteilester von Alkoholen der Kettenlänge C₄ bis C₂₀, bevorzugt C₁₆-C₁₈, die gffs. oxalkyliert sind, in Mengen von 0,5 bis 30, bevorzugt 1 bis 20 Gew-% bezogen auf 100 g eingesetztes Hydrophobierungsmittel enthält.

13. Verfahren nach einem der Ansprüche 10 bis 12, dadurch gekennzeichnet, daß das Hydrophobiermittel Silikonöle; bevorzugt Dimethyl- oder Methylphenylpolysiloxane in Mengen von 0,5 bis 80, bevorzugt 1 bis 10 Gew.-%, bezogen auf 100 g eingesetztes Hydrophobiermittel enthält.

14. Verfahren nach einem der Ansprüche 10 bis 13, dadurch gekennzeichnet, daß das Hydrophobiermittel 0,5 bis 50 bevorzugt 2 bis 20 Gew.-% bezogen auf 100 g Hydrophobiermittel, Ölsäuresarkosid enthält.

15. Verfahren nach einem der Ansprüche 10 bis 14, dadurch gekennzeichnet, daß das Hydrophobiermittel Paraffine und/oder Chlorparaffine enthält.

## Claims

1. A process for the production of compounds of the general formula I 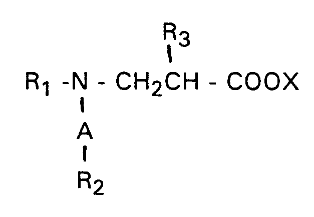 wherein
R₁ is a saturated or unsaturated alkyl radical or alkoxyalkyl radical with 1-22 carbon atoms, preferably 12-18 carbon atoms,
R₂ is an alkyl radical with 1-18 carbon atoms, the oleoyl radical, a saturated or unsaturated carboxyalkyl radical with 3-4 carbon atoms, a carboxyphenyl radical, a carboxyl radical, or a toluyl radical,
R₃ is hydrogen or CH₃, and
X is hydrogen, alkali metal, alkaline-earth metal, ammonium, alkylammonium or alkanolammonium,
and
A stands for 〉C=O, 〉SO₂, -CONH-, or an alkylene radical with 0-3 C-atoms,
with the proviso that
1) if A stands for an alkylene radical with 0-3 C-atoms, R₂ cannot mean an alkyl radical with 1-18 C-atoms or the oleoyl radical,
2) if A stands for 〉SO₂ or -CONH-, R₂ cannot be a saturated or unsaturated carboxyalkyl radical with 3-4 C-atoms, or a carboxyphenyl radical, or a carboxyl radical,
3) if A stands for 〉C=O, R₂ cannot be a carboxyl radical,
which process is characterized in that alkyl amines of the formula R₁-NH₂, wherein R₁ has the above meaning, are added to (meth)-acrylic acid at temperatures of 40 - 130°C, and that the resultant N-alkylaminopropionic acids are reacted with carboxylic acid anhydrides, carboxylic acid chlorides, sulfonic acid chlorides, isocyanates, halogenated carboxylic acids, or (meth)-acrylic acids and are then optionally neutralized, at least in part.

2. The process according to claim 1 characterized in that the addition of alkylamines to (meth)acrylic acid is carried out at temperatures of 70° - 100°C.

3. The process according to claims 1 and 2 characterized in that alkylamines and methacrylic acid are reacted in equimolar quantities.

4. The process according to any one of claims 1 to 3 characterized in that the reaction is carried out under pressure, if alkylamines which boil below the reaction temperatures are used.

5. The process according to any one of claims 1 to 4 characterized in that the N-alkylaminopropionic acids produced in the first step are reacted with carboxylic acid anhydrides, carboxylic acid chlorides, sulfonic acid chlorides, isocyanates, haloacid, or (meth-)acrylic acids at temperatures of 50 to 100°C.

6. The process according to any one of claims 1 to 5 characterized in that carboxylic acid anhydride, carboxylic acid chloride, sulfonic acid chloride, isocyanate, halogenated carboxylic acid, or (meth-)acrylic acids are used in an equimolar ratio or in slight excess, preferably up to 5%, relative to the N-alkylaminopropionic acid.

7. The process according to any one of claims 1 to 6 characterized in that acid-binding agents, preferably powdery sodium carbonate or tertiary amines, are added, if carboxylic acid chlorides, sulfonic acid chlorides, or haloacids are used for the second stage of the reaction.

8. The process according to any one of claims 1 to 7 characterized in that the neutralization is carried out with organic bases, preferably alkylamines and alkanolamines, in particular ethanolamines or propanolamines.

9. N-alkyl-N(2-carboxyethyl)sulfonamides or ureas of the general formula I 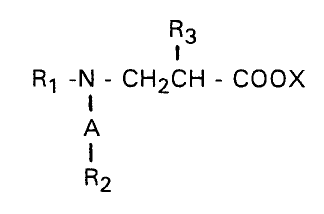 wherein R₁, R₂, R₃, and X have the meaning as stated in claim 1, characterized in that A stands for SO₂ = or -CONH-, with the proviso that
R₂ cannot be an alkyl radical with 1 - 18 C-atoms, a saturated or unsaturated carboxyalkyl radical with 3 - 4 C-atoms, a carboxyphenyl radical, a carboxyl radical, or a toluyl radical.

10. A process for waterproofing leather and furs characterized in that compounds of the general formula I 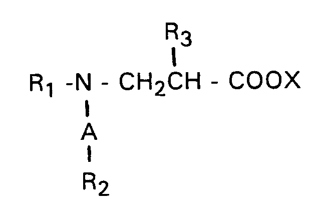 wherein
R₁ is a saturated or unsaturated alkyl radical or alkoxyalkyl radical with 1-22 carbon atoms, preferably 12-18 carbon atoms,
R₂ is an alkyl radical with 1-18 carbon atoms, the oleoyl radical, a saturated or unsaturated carboxyalkyl radical with 3-4 carbon atoms, a carboxyphenyl radical, a carboxyl radical, or a toluyl radical,
R₃ is hydrogen or CH₃, and
X is hydrogen, alkali metal, alkaline-earth metal, ammonium, alkylammonium or alkanolammonium,
and
A stands for 〉C = O, 〉SO₂, -CONH-, or an alkylene radical with 0-3 C-atoms,
with the proviso that
1) if A stands for an alkylene radical with 0-3 C-atoms, R₂ cannot mean an alkyl radical with 1-18 C-atoms or the oleoyl radical,
2) if A stands for 〉SO₂ or -CONH-, R₂ cannot be a saturated or unsaturated carboxyalkyl radical with 3-4 C-atoms, or a carboxyphenyl radical, or a carboxyl radical,
3) if A stands for 〉C=O, R₂ cannot be a carboxyl radical,
are allowed to react with the leather or furs to be treated, either during or after retanning, in an amount of 0.1 - 25% by weight, preferably 1 to 15% by weight, relative to the shaved weight of the leather or the wet weight of the furs, at a pH of 4 to 9, that the pH is subsequently set to the range from 3.5 to 5.0, and that after the treatment fixation is optionally effected with a bivalent, trivalent or tetravalent metallic salt, preferably in aqueous solution.

11. The process according to claim 10 characterized in that a product is used for the waterproofing that comprises 5-80, preferably 10-70%-wt. of compounds of the general formula I, preferably in at least partially neutralized form, optionally 20-70, preferably 30-60%-wt. of hydrocarbons, optionally solubilizers, and water as remainder to 100%.

12. The process according to any one of claims 10 to 11 characterized in that the employed waterproofing agent comprises phosphoric acid partial esters of optionally oxalkylated alcohols of the chain length C₄ to C₂₀, preferably C₁₆-C₁₈, in amounts of 0.5 to 30, preferably 1 to 20%-wt., relative to 100 g of the waterproofing agent that is used.

13. The process according to any one of claims 10 to 12 characterized in that the waterproofing agent comprises silicone oils, preferably dimethyl or methylphenyl polysiloxanes, in amounts of 0.5 to 80, preferably 1 to 10%-wt., relative to 100 g of the waterproofing agent that is used.

14. The process according to any one of claims 10 to 13 characterized in that the waterproofing agent comprises 0.5 to 50, preferably 2 to 20%-wt. of oleic acid sarcoside, relative to 100 g of the waterproofing agent.

15. The process according to any one of claims 10 to 14 characterized in that the waterproofing agent comprises paraffins and/or chloroparaffins.

## Revendications

1. Procédé de préparation de composés de la formule générale 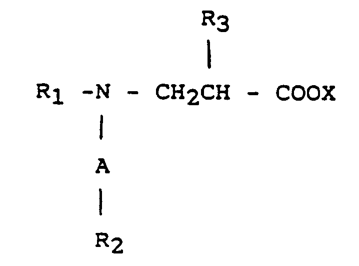 dans laquelle R₁ représente un radical alcoxyalkyle ou un radical alkyle, saturé ou insaturé, comportant de 1 à 22 atomes de carbone, de préférence 12 à 18 atomes de carbone,
R₂ représente un radical alkyle comportant de 1 à 18 atomes de carbone, le radical oléoyle, un radical carboxyalkyle, saturé ou insaturé, comportant de 3 à 4 atomes de carbone, un radical carboxyphényle, un radical carboxyle ou un radical toluyle,
R₃ représente un atome d'hydrogène ou le radical CH₃ et X représente un atome d'hydrogène, un métal alcali, un métal alcalino-terreux, un radical ammonium, alkylammonium ou alcanolammonium et
A représente 〉C = O, 〉SO₂, -CONH- ou un radical alkylène comportant de 0 à 3 atomes de carbone
avec les conditions que
1) lorsque A représente un reste alkylène à 0-3 atomes de carbone, R₂ ne représente ni un radical alkyle à 1-18 atomes de carbone, ni le radical oléyle,
2) lorsque A représente 〉SO₂ ou -CONH-, R₂ ne représente ni un radical carboxyalkyle saturé ou insaturé, ni un radical carboxyphényle, ni un radical carboxyle,
3) lorsque A représente 〉C=O, R₂ ne représente pas de radical carboxyle,
caractérisé en ce que l'on fixe par addition des alkylamines de la formule R₁-NH₂ dans laquelle R₁ possède les significations qui lui ont été attribuées ci-dessus, sur de l'acide (méth)acrylique, à des températures de 40 à 130°C et on fait réagir les acides N-alkylaminopropioniques ainsi formés sur des anhydrides d'acides carboxyliques, des chlorures d'acides carboxyliques, des chlorures d'acides sulfoniques, des isocyanates, des acides halogénocarboxyliques ou des acides (méth)acryliques et on neutralise éventuellement ensuite au moins partiellement les produits obtenus.

2. Procédé suivant la revendication 1, caractérisé en ce que l'addition d'alkylamines sur l'acide (méth)acrylique s'entreprend à des températures de 70 à 100°C.

3. Procédé suivant les revendications 1 et 2, caractérisé en ce que l'on fait réagir les alkylamines et l'acide méthacrylique en proportions équimolaires.

4. Procédé suivant l'une des revendications 1 à 3, caractérisé en ce que l'on entreprend la réaction sous pression lors de l'utilisation d'alkylamine à points d'ébullition inférieurs aux températures réactionnelles.

5. Procédé suivant l'une des revendications 1 à 4, caractérisé en ce que l'on fait réagir les acides N-alkylaminopropioniques formés au cours de la première étape à des températures de 50 à 100°C sur des anhydrides d'acides carboxyliques, des chlorures d'acides carboxyliques, des chlorures d'acides sulfoniques, des isocyanates, des acides halogénocarboxyliques ou des acides (méth)acryliques.

6. Procédé suivant l'une des revendications 1 à 5, caractérisé en ce que l'on utilise un anhydride d'acide carboxylique, un chlorure d'acide carboxylique, un chlorure d'acide sulfonique, un isocyanate, un acide halogénocarboxylique ou des acides (méth)acryliques en une proportion équimolaire ou en un faible excès, allant de préférence jusqu'à 5%, par rapport à l'acide N-alkylaminopropionique.

7. Procédé suivant l'une des revendications 1 à 6, caractérisé en ce que lors de l'emploi de chlorures d'acides carboxyliques, de chlorures d'acides sulfoniques ou d'acides halogénocarboxyliques pour la seconde étape de la réaction, on ajoute des agents qui fixent les acides, de préférence le carbonate de sodium pulvérulent ou des amines tertiaires.

8. Procédé suivant l'une des revendications 1 à 7, caractérisé en ce que l'on entreprend la neutralisation avec des bases organiques, de préférence des alkylamines et des alcanolamines, plus particulièrement des éthanolamines ou des propanolamines.

9. Nouveaux N-alkyl-N-(2-carboxyéthyl)sulfonamides ou urées de la formule générale I 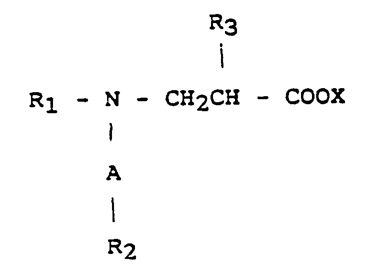 dans laquelle R₁, R₂, R₃ et X possèdent les significations qui leur ont été attribuées dans la revendication 1, caractérisé en ce que A représente SO₂= ou -CONH-, avec la condition que
R₂ ne représente ni radical alkyle à 1-18 atomes de carbone, ni radical carboxyalkyle à 3-4 atomes de carbone, ni radical carboxyphényle, ni radical carboxyle et ni radical toluyle.

10. Procédé pour l'hydrophobisation du cuir et de fourrures, caractérisé en ce qu'on laisse agir des composés de la formule générale 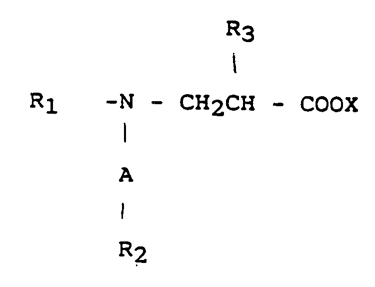 dans laquelle R₁ représente un radical alcoxyalkyle ou un radical alkyle, saturé ou insaturé, comportant de 1 à 22 atomes de carbone, de préférence 12 à 18 atomes de carbone,
R₂ représente un radical alkyle comportant de 1 à 18 atomes de carbone, le radical oléoyle, un radical carboxyalkyle, saturé ou insaturé, comportant de 3 à 4 atomes de carbone, un radical carboxyphényle, un radical carboxyle ou un radical toluyle,
R₃ représente un atome d'hydrogène ou le radical CH₃ et X représente un atome d'hydrogène, un métal alcali, un métal alcalino-terreux, un radical ammonium, alkylammonium ou alcanolammonium et
A représente 〉C = O, 〉SO₂, -CONH- ou un radical alkylène comportant de 0 à 3 atomes de carbone
avec les conditions que
1) lorsque A représente un reste alkylène à 0-3 atomes de carbone, R₂ ne représente ni un radical alkyle à 1-18 atomes de carbone, ni le radical oléyle,
2) lorsque A représente 〉SO₂ ou -CONH-, R₂ ne représente ni un radical carboxyalkyle saturé ou insaturé, ni un radical carboxyphényle, ni un radical carboxyle,
3) lorsque A représente 〉C=O, R₂ ne représente pas de radical carboxyle,
en une proportion de 0,1 à 25% en poids, de préférence 1 à 15% en poids, par rapport au poids du cuir dérayé ou du poids des peaux humides, à des valeurs de pH de 4 à 9, sur le cuire ou les peaux à traiter, pendant ou après le retannage, puis on en règle la valeur du pH dans la plage des 3,5 à 5,0 et, après le traitement, on les fixe éventuellement avec un sel de métal bi, tri ou quadrivalent, de préférence en solution aqueuse.

11. Procédé suivant la revendication 10, caractérisé en ce qu'en vue de l'hydrophobisation, on met en oeuvre un produit qui contient de 5 à 80% en poids, de préférence 10 à 70% en poids, de composés de la formule générale I, de préférence sous une forme au moins partiellement neutralisée, éventuellement 20 à 70% en poids, de préférence 30 à 60% en poids, d'hydrocarbures, éventuellement des agents solubilisants, le reste étant constitué d'eau jusqu'à atteindre la proportion de 100%.

12. Procédé suivant l'une des revendications 10 et 11, caractérisé en ce que l'agent d'hydrophobisation mis en oeuvre contient des esters partiels de l'acide phosphorique d'alcools d'une longueur de chaîne de C₄ à C₂₀, de préférence de C₁₆ à C₁₈, qui sont éventuellement oxalkylés, en proportions de 0,5 à 30, de préférence 1 à 20, % en poids, par rapport à 100 g de l'agent d'hydrophobisation utilisé.

13. Procédé suivant l'une des revendications 10 à 12, caractérisé en ce que l'agent d'hydrophobisation contient des huiles siliconées, de préférence du diméthyl- ou du méthylphénylpolysiloxanne, en proportions qui varient de 0,5 à 80% en poids, de préférence 1 à 10% en poids, par rapport à 100 g de l'agent d'hydrophobisation utilisé.

14. Procédé suivant l'une des revendications 10 à 13, caractérisé en ce que l'agent d'hydrophobisation contient de 0,5 à 50% en poids, de préférence de 2 à 20% en poids, de sarcoside de l'acide oléique, par rapport à 100 g d'agent d'hydrophobisation.

15. Procédé suivant l'une des revendications 10 à 14, caractérisé en ce que l'agent d'hydrophobisation contient des paraffines et/ou des chloroparaffines.
